# EUROPÄISCHE PATENTSCHRIFT

(11) **EP 3 120 899 B1**
(45) Veröffentlichungstag und Bekanntmachung des Hinweises auf die Patenterteilung: **12.07.2023**
(21) Anmeldenummer: 16171975.2
(22) Anmeldetag: 30.05.2016
(51) Int. Cl.: A62B 99/00, B08B 3/04, B08B 9/00, B08B 11/02, F26B 21/00, F26B 25/00, G01M 3/02, F26B 25/06

(54) **VERFAHREN UND SYSTEM ZUR PFLEGE MINDESTENS EINES SCHUTZANZUGS**
METHOD AND SYSTEM FOR MAINTAINING AT LEAST ONE PROTECTIVE SUIT
PROCEDE ET SYSTEME D'ENTRETIEN D'AU MOINS UN VETEMENT DE PROTECTION

(30) Priorität: 03.06.2015 DE 102015108857
(43) Veröffentlichungstag der Anmeldung: 25.01.2017
(73) Patentinhaber: Rud. Prey GmbH & Co. KG, 24113 Kiel (DE)
(72) Erfinder: Prey, Thomas, 24105 Kiel (DE)
(74) Vertreter: Hauck Patentanwaltspartnerschaft mbB

(56) Entgegenhaltungen:
- WO-A1-93/09418
- DE-U1- 29 822 172
- US-A1- 2001 049 883

## Beschreibung

Die Erfindung betrifft ein Verfahren und ein System zur Pflege mindestens eines Schutzanzugs, insbesondere eines Chemikalienschutzanzugs. Chemikalienschutzanzüge (CSA) sind Teil der persönlichen Schutzausrüstung (PSA) der Feuerwehr und anderer Rettungsmannschaften. Sie schützen den Verwender vor äußeren Einflüssen und ermöglichen ein zeitlich begrenztes Arbeiten in durch Chemikalien kontaminierter Umgebung. Durch das Verwenden eines Chemikalienschutzanzugs wird die Aufnahme von schädlichen Stoffen und Gasen über die Haut und Körperöffnungen verhindert. Chemikalienschutzanzüge werden in verschiedene Typen unterteilt (nach DIN 943-1, 943-2, 14605, 13982-1, 13034):
Typ 1 - "Gasdichter" Chemikalienschutzanzug [DIN 943-1 und 943-2]
   Typ 1a-ET - "Gasdichter" Chemikalienschutzanzug:
      Gasdichter Chemikalienschutzanzug für die Verwendung durch Notfallteams mit einer im Chemikalienschutzanzug getragenen umgebungsluftunabhängigen Atemluftversorgung, z.B. einem Behältergerät mit Druckluft (Pressluftatmer).
   Typ 1b-ET - "Gasdichter" Chemikalienschutzanzug:
      Gasdichter Chemikalienschutzanzug für die Verwendung durch Notfallteams mit außerhalb des Chemikalienschutzanzugs getragener Atemluftversorgung, z.B. einem Behältergerät mit Druckluft (Pressluftatmer).
   Typ 1c - "Gasdichter" Chemikalienschutzanzug mit einer Atemluftversorgung mit Überdruck, z.B. aus externen Leitungen.
Typ 2 - "Nicht-Gasdichter" Chemikalienschutzanzug [DIN 943-1] mit einer Atemluftversorgung mir Überdruck.
Typ 3 - "Flüssigkeitsdichter" Chemikalienschutzanzug [DIN 14605]
Typ 4 - "Spraydichter" Chemikalienschutzanzug [DIN 14605]
Typ 5 - "Schwerstaubdichter" Chemikalienschutzanzug [DIN 13982-1]
Typ 6 - "Eingeschränkt gegen flüssige Chemikalien schützender" Chemikalienschutzanzug [DIN 13034].

Bei Chemikalienschutzanzügen der Typen 1 und 2 wird zwischen Anzügen für den begrenzten Einsatz (Einweg) und den wiederverwendbaren Einsatz unterschieden. Nach geltenden Vorschriften für die persönliche Schutzausrüstung der Feuerwehr sind ausschließlich Chemikalienschutzanzüge des Typs 1a-ET und 1b-ET für den Einsatz zugelassen (ET: Emergency Team). Die vorliegende Erfindung befasst sich vorzugsweise mit wiederverwendbaren Chemikalienschutzanzügen der Typen 1a-ET und 1b-ET.

Wiederverwendbare Chemikalienschutzanzüge müssen gepflegt werden. Die Pflege umfasst regelmäßig eine Reinigung, Desinfektion und Trocknung des Chemikalienschutzanzugs von innen und außen, eine Sichtprobe auf Beschädigungen sowie eine Dichtigkeitsprüfung des Anzuges und der Ausatemvorrichtung (Abluftvorrichtung und Ausatemventilöffnung(en)). Das Nachfetten des die Zugangsöffnung für einen Verwender bildenden Reißverschlusses und das Aufbringen einer Antibeschlagslösung auf die Sichtscheibe gehören ebenfalls zur regemäßigen Pflege der Anzüge. Die aufgezählten Pflegearbeiten sind nach jedem Einsatz oder jeder Übung, bei der der CSA verwendet wurde-, durchzuführen. Die Dichtigkeits- und Ausatemventilprüfung ist, falls der Anzug nicht verwendet wurde, in herstellerspezifischen Intervallen halbjährlich bis nach spätestens drei Jahren durchzuführen. Durch Einsätze kontaminierte, verschmutzte oder verunreinigte Chemikalienschutzanzüge werden bereits am Einsatzort dekontaminiert (Entfernen von gefährlichen Verunreinigung) und luftdicht in speziellen Behältnissen (z.B. Überfässern oder Kontaminationssäcken) verpackt und gekennzeichnet (Einsatz, Ort, Art der Kontamination). Stark kontaminierte Anzüge, die mit giftigen und nicht wasserlöslichen Stoffen kontaminiert wurden, sind auszumustern und fachgerecht zu entsorgen.

Die erforderlichen Pflegearbeiten werden in einer dafür speziell ausgestatteten Atemschutzwerkstatt der Feuerwehr durchgeführt. Vor der Pflege der Chemikalienschutzanzüge sind diese auf mögliche Restkontaminationen zu überprüfen.

In jedem Fall ist bei der Pflege der CSA eine leichte Schutzausrüstung von einem Gerätewart oder anderem geschulten Fachpersonal für die Wartung der Schutzausrüstung zu tragen. Neben chemischen Verunreinigungen können auch Verschmutzungen durch Öle, Sande, biologische Abfälle und andere Flüssigkeiten und Feststoffe an der Außenschicht des CSA haften. Die Verunreinigungen im Inneren der Anzüge bestehen lediglich aus Verunreinigungen durch Keime, hervorgerufen durch Schweiß, Ausatemluft, Hautschuppen und Haaren des Verwenders.

Bei Übungen können Übungsanzüge oder (ausrangierte) Einsatzanzüge verwendet werden. Hierbei muss jedoch eine Kontamination mit Gefahrstoffen ausgeschlossen werden, um die Pflege ohne weitere Vorsichtmaßnahmen durchzuführen.

Chemikalienschutzanzüge sind gemäß den Herstellerangaben bzw. den Dienstvorschriften zu reinigen. Bekannt ist eine manuelle Reinigung, bei der der Chemikalienschutzanzug zunächst unter Zuhilfenahme weicher Schwämme oder Bürsten und eines milden Feinwaschmittels im geschlossenen Zustand von außen von allen Seiten gereinigt wird. Nachdem der Anzug mit klarem Wasser abgespült wurde, erfolgt die Innenreinigung. Auch bekannt ist eine Reinigung in Industriewaschmaschinen.

Im Unterschied zu den beiden vorgenannten Verfahren erlauben einige CSA-Pflegeanlagen die gleichzeitige Reinigung mehrerer Chemikalienschutzanzüge. Erfolgt eine solche Reinigung mehrerer Chemikalienschutzanzüge in einer Industriewaschmaschine, ist ein den Herstellangaben entsprechendes Reinigungsprogramm zu wählen. Die Industriewaschmaschine ähnelt dabei einer Haushaltswaschmaschine, ist jedoch erheblich größer dimensioniert. Sie ermöglicht in einem Waschgang allerdings nur die Reinigung und Desinfektion eines Chemikalienschutzanzugs. Durch die Verwendung zusätzlichen Zubehörs können auch Atemschutzmasken gereinigt und desinfiziert werden.

Nach der Reinigung werden die Schutzanzüge gespült und im nächsten Schritt desinfiziert. Die Wahl des zu verwenden Desinfektionsmittels ist den Herstellerangaben zu entnehmen. Bei der manuellen Desinfektion kann mit Hilfe einer Sprühlanze das Desinfektionsmittel innen und außen aufgetragen werden. In Industriewaschmaschinen und CSA-Pflegeanlagen kann die Desinfektion mit Hilfe eines Desinfektionsvollwaschmittels durchgeführt werden.

Die Trocknung der CSA kann je nach Hersteller und Typ an der Luft, im Trockenschrank oder mit Hilfe einer Trocknungsanlage bei Temperaturen bis zu 50°C (Herstellerangaben sind zu beachten) durchgeführt werden. Dabei ist darauf zu achten, dass die CSA nicht der direkten Sonne- bzw. Wärmebestrahlung ausgesetzt sind. Hierdurch sollen Beschädigungen am Material vermieden werden.

Nach einer ersten Sichtprüfung auf optische Mängel folgen die Dichtigkeitsprüfung und die Ausatemventilprüfung. Die Prüfung der Gasdichtigkeit eines Chemikalienschutzanzugs ist gemäß BGG/GUV-G 9102 nach jeder Benutzung, Übung bzw. mindestens einmal jährlich durchzuführen. Das Prüfverfahren ermittelt die Dichtigkeit des Chemikalienschutzanzugs von innen nach außen, obwohl die Gefahr einer Undichtigkeit in der praktischen Anwendung in der Regel von außen nach innen besteht. Es gilt jedoch regelmäßig, dass die Dichtigkeit von innen nach außen gleich der Dichtigkeit von außen nach innen ist. Die Dichtigkeitsprüfung erfolgt über eine vom Hersteller festgelegte Prüföffnung (Abluftvorrichtung, Ausatemventil bzw. Prüfanschluss) und ist entsprechend der DIN-EN 464 oder gemäß Herstellerangabe auszuführen.

Zur Pflege von Chemikalienschutzanzügen sind offene und geschlossene Pflegesysteme bekannt. Bei einem offenen Pflegesystem ist ein Multifunktionsgestell vorgesehen, auf den der zu pflegende Chemikalienschutzanzug aufgezogen werden muss. Das Multifunktionsgestell umfasst mehrere Lanzen oder dergleichen, die unter anderem in die Bein- und Armöffnungen des Chemikalienschutzanzugs einzuführen sind. Sie umfassen Medienauslässe für die Pflege, beispielsweise für ein Reinigungsmittel. In einer dezentralen Steuereinheit sind die medienführenden Aggregate, wie Pumpen, Gebläse, Heizsysteme und Programmsteuerung untergebracht. Von der dezentralen Steuereinheit aus werden die unterschiedlichen Medien, beispielsweise Reinigungs- und Desinfektionslösungen sowie Trocknungsluft, über Schlauchverbindungen durch den zu diesem Zweck teilweise geöffneten Reißverschluss in den auf dem Multifunktionsgestell aufgezogenen Chemikalienschutzanzug eingeleitet. Der Chemikalienschutzanzug befindet sich auf dem Multifunktionsgestell kopfüber und die über den teilweise geöffneten Reißverschluss in den Chemikalienschutzanzug eingeleiteten Medien werden nach ihrer Verteilung in dem Anzug über den teilweise geöffneten Reißverschluss wieder abgeführt, wobei Luft selbsttätig über den Reißverschluss entweicht und flüssige Medien abgepumpt werden. Auf diese Weise ist es möglich, mehrere Chemikalienschutzanzüge von innen zu reinigen, zu desinfizieren und zu trocknen. Darüber hinaus muss von außen eine manuelle Reinigung und Desinfektion der Anzüge erfolgen.

Geschlossene Pflegesysteme verwenden für den inneren Pflegeprozess des Chemikalienschutzanzugs das gleiche Verfahren wie oben zu dem offenen Pflegesystem erläutert. Geschlossene Pflegesysteme besitzen jedoch eine spritzwasserdichte Pflegekabine, in der über entsprechend positionierte Düsen zusätzlich eine Außenreinigung, -desinfektion und -trocknung des Chemikalienschutzanzugs erfolgt.

Die bekannten Pflegesysteme ermöglichen somit eine teilautomatisierte Reinigung, Desinfektion, Spülung und Trocknung der Chemikalienschutzanzüge. Allerdings erfordern die bekannten Pflegesysteme zusätzlich einen erheblichen manuellen Aufwand. Insbesondere das Auffädeln der zu reinigenden Chemikalienschutzanzüge auf das Multifunktionsgestell ist zeitaufwendig und körperlich anstrengend. Darüber hinaus ist eine automatisierte Dichtigkeitsprüfung nicht möglich, da der erforderliche Druckaufbau im Inneren des Chemikalienschutzanzugs in dem für die Reinigung erforderlichen, teilweise geöffneten Zustand des Reißverschlusses nicht erfolgen kann. Daher ist bei den bekannten Pflegesystemen nach Durchführung der Pflege eine manuelle Dichtigkeitsprüfung durch den Gerätewart erforderlich. Das entsprechende Innendruckprüfverfahren regelt die DIN EN 464. Hierdurch entsteht ein erheblicher zeitlicher Mehraufwand für den Gerätewart. Darüber hinaus ist auch die manuelle Druckprüfung körperlich anstrengend.

Aus US 2001/0 049 883 A1 sind eine Vorrichtung und ein Verfahren zum Trocknen von vorzugsweise Trockenanzügen bekannt. Dabei werden Medienzuführungslanzen in den Anzug eingeführt, beispielsweise durch eine Halsöffnung. Über die Medienzuführungslanzen zugeführtes Trocknungsgas wird in das Innere des Anzugs eingeführt und beispielsweise über Armöffnungen abgeführt. Dabei können auch chemische Substanzen, zum Beispiel antibakterielle Mittel, in das Innere des Anzugs eingeführt werden.

Aus WO 93/09418 A1 sind eine Vorrichtung und ein Verfahren zur Leckageprüfung eines Anzugs bekannt. Der Anzug wird auf ein Multifunktionsgestell mit in die Extremitäten des Anzugs führenden Medienzuführungslanzen aufgezogen und es wird eine Mischung aus Wasser und Druckluft über die Medienzuführungslanzen in den Anzug eingesprüht. Der Anzug wird dann optisch auf Leckagen untersucht.

Aus DE 298 22 172 U1 ist ein Kabinensystem zum Behandeln von Schutzanzügen für Katastrophenfälle bekannt mit einer Aufhängung für Kleiderbügel, wobei die Kleiderbügel eine Innenbedüsung sowie eine Heißluftzufuhr aufweisen. Der Kleiderbügel ist als Multifunktionsgestell ausgebildet, auf das ein Anzug aufgezogen werden kann, wobei Medienzuführungslanzen in die Extremitäten des Anzugs eingeführt werden.

Ausgehend von dem erläuterten Stand der Technik liegt der Erfindung daher die Aufgabe zugrunde, ein Verfahren und ein System zur Pflege mindestens eines Schutzanzugs bereitzustellen, mit denen die Pflege von Schutzanzügen schneller und weniger aufwendig erfolgen kann.

Die Erfindung löst die Aufgabe durch die unabhängigen Ansprüche 1 und 9. Vorteilhafte Ausgestaltungen finden sich in den abhängigen Ansprüchen, der Beschreibung und den Figuren.

Die Erfindung löst die Aufgabe einerseits durch ein Verfahren zur Pflege mindestens eines Schutzanzugs, insbesondere eines Chemikalienschutzanzugs, umfassend die Schritte:
a) mindestens ein Pflegeadapter wird mit mindestens einer Öffnung des Schutzanzugs lösbar und dichtend verbunden,
b) über den mindestens einen Pflegeadapter wird ein Reinigungs- und Desinfektionsfluid in das Innere des Schutzanzugs geleitet und das in das Innere des Schutzanzugs geleitete Reinigungs- und Desinfektionsfluid aus dem Schutzanzug abgeführt,
c) über den mindestens einen Pflegeadapter wird ein Trocknungsgas zur Trocknung des Schutzanzugs in das Innere des Schutzanzugs geleitet und das in das Innere des Schutzanzugs geleitete Trocknungsgas aus dem Schutzanzug abgeführt,
d) der mindestens eine Pflegeadapter wird von der mindestens einen Öffnung des Schutzanzugs getrennt.

Außerdem löst die Erfindung die Aufgabe durch ein System zur Pflege mindestens eines Schutzanzugs, insbesondere eines Chemikalienschutzanzugs, umfassend:
- mindestens einen Pflegeadapter, der lösbar und dichtend mit mindestens einer Öffnung des Schutzanzugs verbindbar ist,
- eine mit dem mindestens einen Pflegeadapter verbindbare Reinigungs- und Desinfektionsfluidzuführeinrichtung zum Einleiten eines Reinigungs- und Desinfektionsfluids in das Innere des Schutzanzugs und zum Abführen des in das Innere des Schutzanzugs geleiteten Reinigungs- und Desinfektionsfluids aus dem Schutzanzug,
- eine mit dem mindestens einen Pflegeadapter verbindbare Trocknungsgaszuführeinrichtung zum Einleiten eines Trocknungsgases in das Innere des Schutzanzugs und zum Abführen des in das Innere des Schutzanzugs geleiteten Trocknungsgases aus dem Schutzanzug,
- eine Steuereinrichtung zur Steuerung des Pflegeprozesses.

Bei der Erfindung wird mindestens ein Pflegeadapter mit mindestens einer Öffnung des Schutzanzugs lösbar und dichtend verbunden. Die Medienzuführung in das Innere des Schutzanzugs, insbesondere das Zuführen eines Reinigungs- und Desinfektionsfluids und eines Trocknungsgases, sowie das Abführen der Medien aus dem Inneren des Schutzanzugs erfolgt über den mindestens einen dichtend und lösbar mit der mindestens einen Öffnung des Schutzanzugs verbundenen Pflegeadapter. Der Schutzanzug kann somit während der Pflege, insbesondere während des Einleitens und Abführens des Reinigungs- und Desinfektionsfluids sowie des Einleitens und Abführens des Trocknungsgases, geschlossen sein. Insbesondere kann ein eine Zugangsöffnung für eine Person in den Schutzanzug bildender Reißverschluss des Schutzanzugs im Gegensatz zu dem erläuterten Stand der Technik geschlossen sein, da die Medienzuführung und -abführung über den mindestens einen dichtend mit mindestens einer Öffnung des Schutzanzugs verbundenen Pflegeadapter erfolgt. Die Pflegemedien können sich somit selbsttätig vollständig in dem Schutzanzug verteilen. Auf Multifunktionsgestelle, insbesondere in die Bein- und Armöffnungen führende Medienzuführungslanzen, kann erfindungsgemäß somit verzichtet werden. Damit entfällt auch das aufwendige Aufziehen des Schutzanzugs auf ein solches Multifunktionsgestell. Durch den über mindestens einen Pflegeadapter gewährleisteten dichten Zugang in das Innere des im Übrigen geschlossenen Schutzanzugs wird darüber hinaus auch eine Dichtigkeitsprüfung des Schutzanzugs über den mindestens einen Pflegeadapter ermöglicht. Durch eine geeignete Ventilsteuerung wird gewährleistet, dass nacheinander verschiedene Medien in das Innere des Schutzanzugs eingeleitet und aus dem Inneren des Schutzanzugs abgeführt werden können, ohne dass ein Lösen bzw. ein Wechsel des Pflegeadapters oder mindestens eines den mindestens einen Pflegeadapter mit den entsprechenden Medienzuführeinrichtungen verbindenden Schlauchs erforderlich ist. Alle Pflegevorgänge können somit über den vor der Pflege mit der mindestens einen Öffnung des Schutzanzugs lösbar und dicht verbundenen mindestens einen Pflegeadapter und die mit diesem verbundenen Zuführ- und Abführschläuche erfolgen. Sowohl der zeitliche als auch der körperliche Aufwand für einen Gerätewart werden gegenüber konventionellen Pflegeanlagen massiv verringert.

Während der Reinigung des Schutzanzugs wird durch die Ventilsteuerung mit einer geeigneten Sensorik sichergestellt, dass die Temperatur und der Innendruck des Schutzanzugs jeweils in einem vorgegebenen Bereich bleiben.

Der Schutzanzug kann auch bei der Erfindung an den Fußbereichen aufgehängt werden, so dass der Kopfbereich des Schutzanzugs unten hängt. Um eine Zirkulation des Reinigungs- und Desinfektionsfluids sowie des Trocknungsgases bzw. weiterer Pflegemedien im Inneren des Schutzanzugs zu gewährleisten, werden über eine Ablasseinrichtung über den mindestens einen Pflegeadapter das Reinigungs- und Desinfektionsfluid bzw. das Trocknungsgas und ggf. weitere Pflegemedien abgeführt. Während der Pflege kann sich Kondensat im Inneren des Schutzanzug bilden, welches sich in dem üblicherweise unten befindlichen Kopfbereich des Schutzanzugs ansammeln kann, beispielsweise Kondensat aus einem Reinigungsfluid, einer Desinfektionslösung etc.. Dieses Kondensat kann durch Anordnen einer zusätzlichen Absaugvorrichtung im Inneren des Kopfbereichs des Schutzanzugs abgesaugt werden. Die Absaugvorrichtung kann ebenfalls mit dem mindestens einen Pflegeadapter verbunden sein, sodass das abgesaugte Kondensat über den mindestens einen Pflegeadapter abgeführt wird.

Bei dem Trocknungsgas kann es sich beispielsweise um Trocknungsluft, insbesondere Warmluft, handeln. Dazu kann ein Heizgebläse vorgesehen sein, das einen Volumenstrom erzeugt, der durch den mindestens einen Pflegeadapter in das Innere des Schutzanzugs geleitet wird und sich dort ebenso wie das zuvor eingeleitete Reinigungs- und Desinfektionsfluid selbststätig verteilt. Der Begriff Fluid umfasst im Sinne der vorliegenden Anmeldung alle fließfähigen Stoffe unabhängig von ihrem Aggregatzustand. Insbesondere umfasst der Begriff Fluid gasförmige oder flüssige Stoffe oder Stoffe, die sowohl gasförmige als auch flüssige Bestandteile besitzen, wie beispielsweise ein Dampf.

Das erfindungsgemäße System bzw. das erfindungsgemäße Verfahren können eine integrierte Wasserenthärtung für im Rahmen der Pflege genutztes Wasser enthalten. Auch kann eine integrierte Wasseraufbereitung bzw. -wiederverwendung des für die Pflege genutzten Wassers vorgesehen sein. Die Steuereinrichtung des erfindungsgemäßen Systems kann im Zuge der Pflege erzeugte Messdaten automatisch erfassen und protokollieren, so dass auch nachträglich noch die dem jeweiligen Schutzanzug eindeutig zugeordneten Pflegedaten vorliegen und abgerufen werden können.

Zumindest die Verfahrensschritte b) und c) können von einer Steuereinrichtung automatisch durchgeführt werden, insbesondere vollautomatisch. Der zeitliche und manuelle Aufwand für die Pflege wird dadurch weiter verringert. Ein Gerätewart muss lediglich den mindestens einen Pflegeadapter mit der mindestens einen Öffnung verbinden bzw. nach der Pflege wieder von der mindestens einen Öffnung lösen. Alle anderen Pflegeschritte, bis auf die Sichtprüfung und die Ventilprüfung sowie die Reißverschlussbehandlung, können von der Steuereinrichtung automatisch durchgeführt werden.

Erfindungsgemäß ist weiterhin vorgesehen, dass nach dem Verfahrensschritt b) und vor dem Verfahrensschritt c) in einem Verfahrensschritt b1) über den mindestens einen Pflegeadapter ein Druckgas zur Dichtigkeitsprüfung des Schutzanzugs in das Innere des Schutzanzugs geleitet wird bis in dem Schutzanzug ein vorgegebenes Druckniveau herrscht, das in dem Schutzanzug herrschende Druckniveau über einen vorgegebenen Zeitraum gemessen wird, und anschließend das in das Innere des Schutzanzugs geleitete Druckgas über den mindestens einen Pflegeadapter aus dem Schutzanzug abgeführt wird. Bei dem Druckgas kann es sich beispielsweise um Druckluft handeln. Erfindungsgemäß ist weiterhin vorgesehen, dass in dem Verfahrensschritt b1) weiterhin eine Warnmeldung ausgegeben wird, sofern das Druckniveau in dem vorgegebenen Zeitraum unter einen definierten Grenzwert fällt. Die Warnmeldung kann eine Protokollierung umfassen. Ein undichter Schutzanzug kann repariert anschließend erneut geprüft werden, sei dies in der erfindungsgemäßen Weise oder anderweitig, beispielsweise manuell. Auch der Verfahrensschritt b1) kann von der Steuereinrichtung automatisch, insbesondere vollautomatisch, durchgeführt werden. Durch den dichten Anschluss des mindestens einen Pflegeadapters an die mindestens eine Öffnung des Schutzanzugs kann die Druckprüfung, wie bereits erläutert, ebenfalls über den mindestens einen Pflegeadapter erfolgen, über den auch die Reinigung und Trocknung erfolgt.

Für die Dichtigkeitsprüfung des Schutzanzugs kann durch geeignete Steuerung des Druckgases zunächst ein Stabilisierungsdruck eingestellt und für einen vorgegebenen Zeitraum gehalten werden. Anschließend kann durch geeignete Einstellung des zugeführten Druckgases der höhere Prüfdruck eingestellt und der Schutzanzug durch Ansteuerung der Ventilsteuerung geschlossen werden. Für einen vorgegebenen Zeitraum misst die Druckmesseinrichtung in diesem geschlossenen Zustand einen etwaigen Druckabfall im Inneren des Schutzanzugs. Überschreitet dieser Druckabfall einen Grenzwert, erfüllt der Schutzanzug nicht die Dichtigkeitsanforderungen und es wird eine Warnmeldung ausgegeben. Das Prüfergebnis kann durch die Steuereinrichtung automatisch registriert und für eine spätere Überprüfung dokumentiert werden. Für das Ausgeben der Warnmeldung erfolgt eine entsprechende Bewertung des Prüfergebnisses. Es ist auch möglich, dass die Dichtigkeitsprüfeinrichtung in die Steuereinrichtung des erfindungsgemäßen Systems integriert ist.

Die mindestens eine Öffnung des Schutzanzugs kann mindestens eine Ventilöffnung, insbesondere mindestens eine Zuluftventilöffnung und/oder mindestens eine Abluftventilöffnung, des Schutzanzugs sein. Chemikalienschutzanzüge verfügen in der Regel über eine oder mehrere Abluftventilöffnungen und teilweise über eine Zuluftventilöffnung , wobei die durch den Verwender des Schutzanzugs eingeatmete Luft über die Zuluft(ventil-)öffnung zugeführt und die ausgeatmete Luft über die Abluftventilöffnung(en) abgeführt wird. Abluftventilöffnung(en) sind erforderlich, damit die Ausatemluft keinen Überdruck im Anzug erzeugt.

Wie eingangs erläutert, betrifft die vorliegende Erfindung insbesondere Chemikalienschutzanzüge der Typen 1a-ET und 1b-ET. Anzüge des Typs 1a-ET besitzen eine unter dem Anzug getragene Atemschutzmaske und einen Pressluftatmer. Abluftventilöffnungen sind hier notwendig und vorgeschrieben. Eine Zuluftventilöffnung ist dagegen optional, insbesondere für Atemluft bzw. Klimatisierung. Sofern nur eine Abluftventilöffnung vorhanden ist, muss ein Prüfanschluss am Anzug vorhanden sein. Anzüge des Typs 1b-ET besitzen eine über dem Anzug getragene Atemschutzmaske und einen Pressluftatmer. Anzüge des Typs 1a-ET müssen entsprechend der DIN EN 943-1 über mindestens ein Ausatemventil als Abluftvorrichtung verfügen, damit im CSA kein Überdruck entsteht. Diese Ausatemventile befinden sich in den meisten Fällen (Ausnahme: der Typ1b-ET) im Nacken- oder hinteren Kopfbereich des CSA. Gleiches gilt für die Anzüge des Typs 1b-ET für den Fall, dass das Atemschutzgerät die verbrauchte Atemluft nicht direkt in das Freie abgeben kann. Laut DIN EN 943-1 verfügen Anzüge des Typs 1b-ET über eine Abluftvorrichtung. Sie sind anzuwenden für Anzüge, bei denen Luft von der Atemschutzmaske in den Chemikalienschutzanzug geleitet wird und wenn das Behältergerät außerhalb des Anzuges getragen wird und Luft aus der Druckflasche zur Belüftung in den Anzug geleitet wird. Bei Chemikalienschutzanzügen des Typs 1b ET werden Differenzierungen bezüglich der Maskentypen getroffen. Hier wird zwischen zwei verschiedenen Ausführungen unterschieden:
Ausführung 1:
   - eine in den Anzug voll integrierte Maske
Ausführung 2:
   - - eine separate Maske, die keine feste Verbindung mit dem CSA aufweist.

In besonders einfacher Weise ist es erfindungsgemäß möglich, dass der mindestens eine Pflegeadapter, insbesondere mehrere Pflegeadapter, auch ohne Ausbau der in den Zuluft- bzw. Abluftventilöffnungen angeordneten Ventilmembranen angeschlossen werden kann bzw. können. Es ist erfindungsgemäß aber auch möglich, dass die Ventilmembranen zunächst ausgebaut werden und der mindestens eine Pflegeadapter anschließend mit der Zuluft- und/oder Abluftventilöffnung verbunden wird.

Die Nutzung ohnehin am Schutzanzug vorgesehener Öffnungen für die erfindungsgemäße Pflege führt zu einer weiteren Vereinfachung. Alternativ ist es jedoch auch denkbar, dass die mindestens eine Öffnung eine speziell zu dem Zwecke der Pflege vorgesehene Pflegeöffnung des Schutzanzugs ist. Entsprechend können selbstverständlich auch mehrere derartiger Pflegeöffnungen vorgesehen sein, die erfindungsgemäß mit mehreren Pflegeadaptern verbunden werden können. Es ist auch denkbar, dass es sich bei der mindestens einen Öffnung des Schutzanzugs um eine Visier- bzw. Maskenöffnung handelt, die im Betrieb des Schutzanzugs mit einem entsprechenden Visier bzw. einer Maske verschlossen wird. In diesem Fall kann anstelle des Visiers bzw. der Maske eine Dichtplatte in die Öffnung gesetzt werden, die dann eine oder mehrere geeignete Anschlussöffnungen für den einen oder die mehreren Pflegeadapter besitzt.

Nach einer weiteren Ausgestaltung kann das Reinigungs- und Desinfektionsfluid ein temperierter Reinigungs- und Desinfektionsdampf sein. Der Reinigungs- und Desinfektionsdampf wird durch eine Temperiereinrichtung, ggf. in Verbindung mit einem Temperatursensor, auf ein für die Reinigung vorgegebenes Temperaturniveau gebracht. Bei dieser Ausgestaltung wird anstelle einer konventionellen Zerstäubungstechnik also ein Dampfreinigungsverfahren angewendet. Der Reinigungs- und Desinfektionsdampf verteilt sich besonders gleichmäßig und im Sinne thermischer Schwerkraft selbsttätig in dem Schutzanzug, auch ohne Verwendung eines Multifunktionsgestells. Insbesondere wird der Schutzanzug durch das Zuführen des Reinigungs- und Desinfektionsdampfes und/oder durch Zuführen eines zusätzlichen Druckgases, beispielsweise Druckluft, in im Übrigen verschlossenen Zustand formstabil aufgeblasen. Wie erwähnt, ist für diese Reinigung kein Auffädeln des Anzugs auf ein Multifunktionsgestell, insbesondere kein Einführen von Reinigungsvorrichtungen, wie Reinigungslanzen oder dergleichen, in die Extremitätenaufnahmen des Anzugs erforderlich. Lediglich im Kopfbereich kann eine noch zu erläuternde Absaugvorrichtung für sich sammelndes Kondensat vorgesehen sein.

Bei dem Reinigungs- und Desinfektionsdampf kann es sich nach einer bevorzugten Ausgestaltung um Wasserdampf handeln. Bei der Verwendung eines entsprechend erhitzten Wasserdampfes kann darüber hinaus in besonders einfacher Weise auf ein zusätzliches, beispielsweise chemisches Desinfektionsmittel zur Desinfektion des Innenraums des Schutzanzugs verzichtet werden. Der Reinigungs- und Desinfektionsdampf kann also durch reinen Wasserdampf gebildet sein, der neben einer Reinigungswirkung gleichzeitig die erforderliche Desinfektion bewirkt. So werden im Innenraum befindliche, im Wesentlichen auf den Verwender des Schutzanzugs zurückgehende Keime durch eine ausreichend hohe Temperatur des Wasserdampfes bereits abgetötet. Damit kann in weiter vereinfachender Weise auch auf einen anschließenden Spülvorgang verzichtet werden, der normalerweise erforderlich ist, um ein chemisches Desinfektionsmittel aus dem Inneren des Schutzanzugs wieder zu entfernen.

Durch die homogene Verteilung des Reinigungs- und Desinfektionsdampfes und die damit verbundene Erhöhung der Luftfeuchtigkeit im Inneren des Schutzanzugs kommt es zu einer Kondensation des Reinigungs- und Desinfektionsdampfes am Innenmaterial des Schutzanzugs. Das ablaufende Kondensat erzielt eine reinigende Wirkung der Innenseite des Schutzanzugs. Um die in diesem Zusammenhang in dem üblicherweise während der Pflege nach unten hängenden Kopfteil des Anzuges anfallende Kondensatflüssigkeit zu entfernen, kann der mindestens eine Pflegeadapter weiterhin mit einer Saugleitung verbunden sein, die über einen Kupplungsmechanismus mit einer im Inneren des Kopfteils des Schutzanzugs angeordneten Absaugvorrichtung verbunden ist. Damit kann das Kondensat aus dem Schutzanzug abgesaugt werden.

Alternativ zu der oben erläuterten Dampfreinigung ist erfindungsgemäß auch eine Schaumreinigung und -desinfektion möglich. Dabei wird als Reinigungs- und Desinfektionsfluid ein reinigender und gegebenenfalls desinfizierender Schaum in das Innere des Schutzanzugs eingeleitet, dort verteilt und dann restlos wieder abgeführt.

Insbesondere wenn der Schutzanzug die für ein Abtöten von Keimen im Inneren des Schutzanzugs erforderlichen Temperaturen nicht verträgt, kann dem Reinigungs- und Desinfektionsfluid, beispielsweise einem Wasserdampf, ein gesondertes, beispielsweise chemisches Desinfektionsmittel zugemischt werden. Dann wird nach dem Abführen des in das Innere des Schutzanzugs geleiteten Reinigungs- und Desinfektionsfluids mit dem zugemischten Desinfektionsmittel das Innere des Schutzanzugs mit einem Spülfluid, beispielsweise Wasserdampf, gespült, um das Desinfektionsmittel aus dem Schutzanzug zu entfernen. Das Spülfluid wird dabei über den mindestens einen Pflegeadapter in das Innere des Schutzanzugs geleitet und aus dem Schutzanzug abgeführt. Für das Zumischen des Desinfektionsmittels kann eine Dosiereinrichtung mit einer Dosierpumpe vorgesehen sein. Der Leitung für das Reinigungs- und Desinfektionsfluid wird dann über einen Bypass die benötigte Menge Desinfektionsmittel zugeführt.

Nach einer weiteren Ausgestaltung kann der Schutzanzug zumindest während des Einleitens und des Abführens des Reinigungs- und Desinfektionsfluids durch Einleiten eines Druckgases unter einem die Formstabilität des Schutzanzugs gewährleistenden Ausdehnungsdruck gesetzt werden. Dies kann auch während weiterer Verfahrensschritte erfolgen, insbesondere während eines ggf. erfolgenden Spülens und/oder während der Trocknung. Das Druckgas kann ebenfalls über den mindestens einen Pflegeadapter zugeführt und abgeführt werden. Bei dieser Ausgestaltung wird der Anzug für die Pflege mithilfe von Druckgas, insbesondere Druckluft, unter einen für den Anzug geeigneten Ausdehnungsdruck gesetzt. Dies gewährleistet die homogene Verteilung der in den Anzug eingeführten Pflegemedien und das Erreichen sämtlicher Bereiche des Anzugs durch die Pflegemedien. Das Anzugsmaterial dehnt sich zu seinem vollen Volumen aus und insbesondere bei einer Wasserdampfreinigung kann das anfallende Kondensat ungehindert im Kopfteil des Schutzanzugs zusammenlaufen. Die Zuführung des Druckgases kann über einen Kompressor über dieselbe Leitung erfolgen, wie die Zuführung des Reinigungs- und Desinfektionsfluids, insbesondere eines Wasserdampfes.

Um die insbesondere für die Druckprüfung erforderliche Dichtheit sicherzustellen, wird der Schutzanzug, wie bereits erläutert, während der erfindungsgemäßen Pflege geschlossen. Insbesondere gilt dies für einen eine Zugangsöffnung für eine Person in den Anzug bildenden Reißverschluss des Schutzanzugs.

Nach einer weiteren Ausgestaltung kann vorgesehen sein,
- dass ein erster Pflegeadapter mit einer ersten Öffnung des Schutzanzugs lösbar und dichtend verbunden wird, und ein zweiter Pflegeadapter mit einer zweiten Öffnung des Schutzanzugs lösbar und dichtend verbunden wird,
- dass das Einleiten des Reinigungs- und Desinfektionsfluids über den ersten Pflegeadapter und das Abführen des Reinigungs- und Desinfektionsfluids über den zweiten Pflegeadapter erfolgt, und
- dass das Einleiten des Trocknungsgases über den ersten Pflegeadapter oder den zweiten Pflegeadapter und das Abführen des Trocknungsgases über den zweiten Pflegeadapter oder den ersten Pflegeadapter erfolgt.

Die erste und zweite Öffnung können beispielsweise Ventilöffnungen des Schutzanzugs sein, beispielsweise Zuluft- und Abluftventilöffnungen. Es kann sich aber auch um andere Öffnungen handeln. Das Einleiten des Druckgases kann über den ersten Pflegeadapter oder den zweiten Pflegeadapter erfolgen und das Abführen des Druckgases kann über den zweiten Pflegeadapter oder den ersten Pflegeadapter erfolgen. Das Einleiten von Spülfluid zum Spülen des Schutzanzugs kann über den ersten oder den zweiten Pflegeadapter erfolgen und das Abführen des Spülfluids kann über den zweiten oder den ersten Pflegeadapter erfolgen.

Wie bereits erläutert, stellen die erfindungsgemäßen Pflegeadapter eine dichtende und zugleich lösbare Verbindung zwischen der äußeren Atmosphäre und der Atmosphäre im Inneren des Schutzanzugs sicher. Bei den vorgenannten Ausgestaltungen kann die Zuführung und die Abführung der jeweiligen Medien jeweils über unterschiedliche Pflegeadapter erfolgen. Es ist aber auch möglich, dass für einige oder sämtliche der Pflegemedien die Zuführung und Abführung über denselben Pflegeadapter erfolgt. Wie bereits erläutert, ist erfindungsgemäß eine geeignete Ventilsteuerung vorgesehen, um nacheinander unterschiedliche Medien zu- und abführen zu können, ohne die Pflegeadapter oder mit den Pflegeadaptern verbundene Zuführ- und Abführschläuche lösen zu müssen. Sofern ein Zuführen und Abführen über denselben Pflegeadapter erfolgt, können Schlauch-in-Schlauch-Systeme zum Einsatz kommen.

Nach einer weiteren Ausgestaltung kann die Pflege des Schutzanzugs in einer verschließbaren Pflegekabine erfolgen, wobei die lösbare und dichtende Verbindung des mindestens einen Pflegeadapters mit der mindestens einen Öffnung außerhalb der Pflegekabine erfolgen und der Schutzanzug automatisch in die Pflegekabine verfahren werden kann. Das Verfahren in die Pflegekabine kann insbesondere vollautomatisch erfolgen. Der gepflegte Schutzanzug kann von der Steuereinrichtung nach der Pflege auch wieder automatisch, insbesondere vollautomatisch, aus der Pflegekabine heraus verfahren werden. Die Pflegekabine ist insbesondere spritzwasserdicht. Tropfwasser kann entweichen. Das dichte Verbinden des mindestens einen Pflegeadapters mit der mindestens einen Öffnung vor der Pflege und das Lösen des mindestens einen Pflegeadapters von der mindestens einen Öffnung nach der Pflege kann manuell durch einen Gerätewart außerhalb der Pflegekabine erfolgen. Die Pflegekabine kann vor Beginn und am Ende der Pflege (voll-)automatisch oder manuell geschlossen bzw. geöffnet werden. In der geschlossenen Pflegekabine kann weiterhin insbesondere automatisch eine Reinigung, Desinfektion und Trocknung der Außenseite des jeweiligen Schutzanzugs erfolgen. Die Pflegekabine erlaubt darüber hinaus eine automatische Schwarz-Weiß-Trennung, also eine sichere Trennung zwischen ungereinigten und gereinigten Schutzanzügen. Die Zuführeinrichtung zum Zuführen der zu pflegenden Schutzanzüge in die Pflegekabine (und gegebenenfalls zum Herausfahren der gepflegten Schutzanzüge aus der Pflegekabine) kann Schienen umfassen, auf denen Transportschlitten verschiebbar geführt sind, wobei der oder die Schutzanzüge an den Transportschlitten befestigt, beispielsweise mit ihren Fußteilen aufgehängt werden können. Auf diese Weise können die Schutzanzüge in die Pflegekabine und aus dieser heraus verfahren werden. Auch denkbar ist der Einsatz von Teleskopschienen, an denen die Schutzanzüge befestigt, beispielsweise mit ihren Fußteilen aufgehängt sind, wobei die Teleskopschienen dann zum Einfahren und Herausfahren der Schutzanzüge in die bzw. aus der Pflegekabine eingefahren bzw. herausgefahren werden können. Die Steuerung des Transportschlittens bzw. der Teleskopschienen erfolgt automatisch durch die erfindungsgemäße Zuführeinrichtung, beispielsweise gesteuert durch die Steuereinrichtung.

Nach einer weiteren Ausgestaltung kann vorgesehen sein, dass mit dem erfindungsgemäßen Verfahren bzw. dem erfindungsgemäßen System mehrere Schutzanzüge gleichzeitig gepflegt werden können. Es sind dann entsprechend für jeden der Schutzanzüge ein oder mehrere erfindungsgemäße Pflegeadapter vorgesehen, die jeweils mit einer oder mehreren Öffnungen, zum Beispiel Ventilöffnungen, wie Zuluft- und/oder Abluftventilöffnung(en), der Schutzanzüge verbunden werden. Es ist dann möglich, dass eine gemeinsame Reinigungs- und Desinfektionsfluidzuführeinrichtung, eine gemeinsame Trocknungsgaszuführeinrichtung, eine gemeinsame Dichtigkeitsprüfeinrichtung (mit jeweils einer Druckmesseinrichtung pro Schutzanzug), eine gemeinsame Dosiereinrichtung, eine gemeinsame Spüleinrichtung und/oder eine gemeinsame Formstabilitätsdruckeinrichtung für alle Schutzanzüge vorgesehen sind. Ebenfalls kann eine gemeinsame Steuereinrichtung zur gleichzeitigen Pflege aller Schutzanzüge vorgesehen sein. Es ist aber auch denkbar, dass einzelne oder sämtliche der vorgenannten Einrichtungen mehrfach, insbesondere einmal pro Schutzanzug, vorgesehen sind.

Durch die Erfindung können ein automatisiertes Reinigen, Desinfizieren und Trocknen mit einer automatisierten und DIN- und Herstellergerechten Dichtigkeitsprüfung kombiniert werden. Der zeitliche und körperliche Aufwand für einen Bediener wird deutlich reduziert. Dies gilt insbesondere durch den Verzicht auf ein Multifunktionsgestell für die Innenpflege aufgrund des erfindungsgemäßen Einsatzes von Pflegeadaptern, die an ggf. ohnehin vorhandene Öffnungen des Schutzanzugs direkt angeschlossen werden. Durch den Einsatz einer Dampfreinigung kann unter gewissen Voraussetzungen auf die Verwendung zusätzlicher Reinigungs- bzw. Desinfektionsmittel verzichtet werden. Dadurch wird auch der Wasserverbrauch reduziert.

Ein Ausführungsbeispiel der Erfindung wird nachfolgend anhand von Figuren näher erläutert. Es zeigen schematisch:

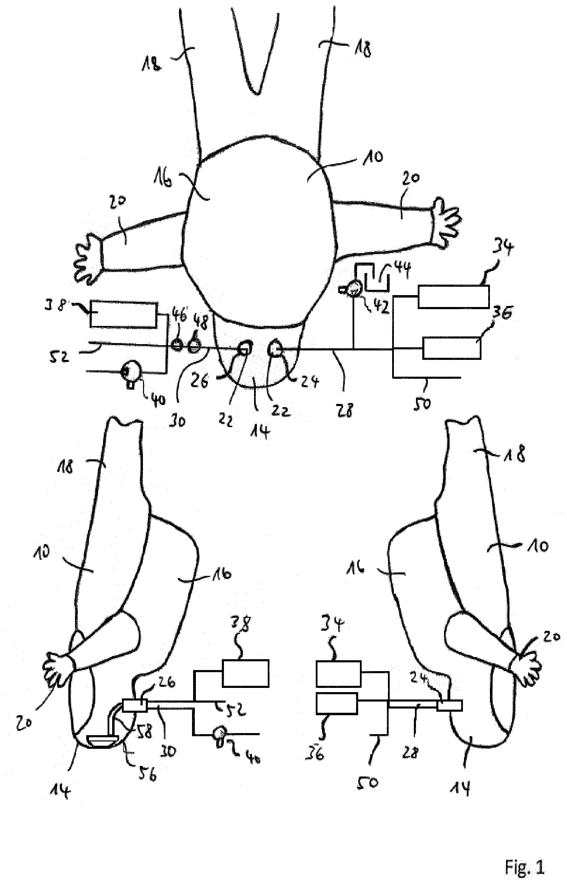

- Figur 1: ein erfindungsgemäßes System zur Pflege eines Chemikalienschutzanzugs in einer ersten Rückansicht im oberen Bildteil, in einer ersten teilweisen Schnittansicht im unteren linken Bildteil und in einer zweiten teilweisen Schnittansicht im unteren rechten Bildteil,
- Figur 2: ein weiteres Detail des in Fig. 1 gezeigten erfindungsgemäßen Systems,
- Figur 3: ein weiteres Detail des in Fig. 1 gezeigten erfindungsgemäßen Systems in einer perspektivischen Ansicht, und
- Figur 4: das Detail aus Figur 3 in einer Rückansicht.

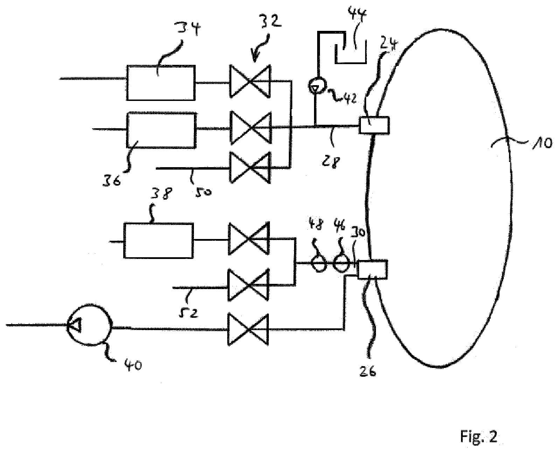

Soweit nichts anderes angegeben ist, bezeichnen in den Figuren gleiche Bezugszeichen gleiche Gegenstände. In den Figuren ist bei dem Bezugszeichen 10 allgemein ein Chemikalienschutzanzug gezeigt, wobei der Chemikalienschutzanzug in Figur 2 lediglich durch eine Ellipse veranschaulicht ist. Der Chemikalienschutzanzug ist in den Figuren im an den Fußabschnitten 12 aufgehängten Zustand gezeigt, so dass ein Kopfabschnitt 14 des Chemikalienschutzanzugs nach unten hängt. Der Chemikalienschutzanzug 10 besitzt darüber hinaus einen Rumpfabschnitt 16 und Beinabschnitte 18 sowie Arm- und Handabschnitte 20. Es handelt sich bei dem Chemikalienschutzanzug beispielsweise um einen Anzug des Typs 1a-ET oder 1b-ET.

An der Rückseite des Kopfabschnitts 14 befinden sich zwei Abluftventilöffnungen 22, wobei in dem gezeigten Beispiel mit einer ersten Abluftventilöffnung 22 ein erster Pflegeadapter 24 lösbar dichtend verbunden ist und mit einer zweiten der Abluftventilöffnungen 22 ein zweiter Pflegeadapter 26 lösbar und dichtend verbunden ist. Ein erster Schlauch 28 ist mit dem ersten Pflegeadapter 24 verbunden und ein zweiter Schlauch 30 ist mit dem zweiten Pflegeadapter 26 verbunden. Über eine anhand von Figur 2 noch näher zu erläuternde Ventilsteuerung 32 können die Pflegeadapter 24, 26 mit unterschiedlichen Medienzuführungen und Medienabführungen verbunden werden. Während der Pflege des Chemikalienschutzanzugs 10 mit dem erfindungsgemäßen System ist ein eine Zugangsöffnung für eine Person bildender Reißverschluss dicht geschlossen.

Bei dem Bezugszeichen 34 ist ein Wasserdampfgenerator einer Reinigungs- und Desinfektionsfluidzuführeinrichtung gezeigt. Bei dem Bezugszeichen 36 ist ein Druckluftkompressor gezeigt, der zu einer Dichtigkeitsprüfeinrichtung des erfindungsgemäßen Systems gehört. Bei dem Bezugszeichen 38 ist ein Heizgebläse einer Trocknungsgaszuführeinrichtung gezeigt und bei dem Bezugszeichen 40 eine Pumpe. Weiterhin ist bei dem Bezugszeichen 42 eine Dosierpumpe gezeigt, die zu einer Dosiereinrichtung des erfindungsgemäßen Systems gehört. Die Dosierpumpe 42 steht mit einem Reservoir 44 für Desinfektionsmittel in Verbindung. Bei dem Bezugszeichen 46 ist ein Drucksensor und bei dem Bezugszeichen 48 ein Temperatursensor des erfindungsgemäßen Systems dargestellt. Insbesondere in Figur 2 ist zu erkennen, dass der Wasserdampfgenerator 34, der Druckluftkompressor 36, das Heizgebläse 38 und die Pumpe 40 jeweils über eine mit einem Ventil der Ventilsteuerung 32 versehene Leitung mit den Schläuchen 28 bzw. 30 verbunden sind. Außerdem sind eine erste Abführleitung 50 und eine zweite Abführleitung 52 vorgesehen, die ebenfalls jeweils über eine mit einem Ventil der Ventilsteuerung 32 versehene Leitung mit dem ersten Schlauch 28 bzw. dem zweiten Schlauch 30 verbunden sind. Es sei darauf hingewiesen, dass die Ventilsteuerung 32 aus Veranschaulichungsgründen nur in Figur 2 dargestellt ist.

Nachfolgend soll die Funktion des erfindungsgemäßen Systems zur Pflege des Chemikalienschutzanzugs erläutert werden:
Zunächst wird der Chemikalienschutzanzug 10 für die Pflege vorbereitet, wobei die Belüftungseinheit, Anbauteile, ein Anti-Fog-Visier, Rückenpolster, Membranen (Scheiben) der Abluftvorrichtung (Ausatemventile) und Abdeckungen der Anzugventile einzeln oder vollständig entfernt werden können und eine Sichtprüfung des Chemikalienschutzanzugs 10 auf offensichtliche Beschädigungen durchgeführt wird. Der Chemikalienschutzanzug 10 wird anschließend oder auch vorher mit oder ohne die erläuterten Anbauteile kopfüber mit dem Fußteil 12 an der Pflegeanlage angeordnet, vorliegend an einer in den Figuren 3 und 4 bei dem Bezugszeichen 54 schematisch gezeigten Schiene. Bei der Schiene 54 kann es sich um eine Teleskopschiene handeln, mit der der Chemikalienschutzanzug 10 automatisch oder manuell in eine und aus einer nicht näher dargestellten Pflegekabine des Pflegesystems gefahren werden kann. Die Pflegekabine kann vor der Pflege automatisch oder manuell verschlossen werden und nach der Pflege automatisch oder manuell geöffnet werden. Für die Steuerung des erfindungsgemäßen Systems einschließlich einer ggf. vorgesehenen Teleskopschiene 54 ist eine nicht näher dargestellte Steuereinrichtung vorgesehen.

Nach dem Aufhängen des Chemikalienschutzanzugs an der Schiene 54 wird beispielsweise manuell durch einen Gerätewart der erste Pflegeadapter 24 mit der ersten Abluftventilöffnung 22 lösbar und dichtend verbunden und der zweite Pflegeadapter 26 mit der zweiten Abluftventilöffnung 22 lösbar und dichtend verbunden. Darüber hinaus wird zum Absaugen von sich im Rahmen der Pflege im Kopfteil 14 ansammelndem Kondensat eine Absaugvorrichtung 56 im Inneren des Kopfteils 14 angeordnet, die über eine Absaugleitung 58 mit dem zweiten Pflegeadapter 26 verbunden ist. Zum Verbinden der Pflegeadapter 24, 26 sowie zum Anordnen der Absaugvorrichtung 56 einschließlich Absaugleitung 58 in dem Chemikalienschutzanzug 10 ist ein eine Zugangsöffnung bildender Reißverschluss des Chemikalienschutzanzugs 10 geöffnet. Dieser wird nach der Installation der genannten Komponenten verschlossen. Anschließend kann der Chemikalienschutzanzug 10 wie erläutert beispielsweise in eine Pflegekabine des Systems verfahren werden.

Es beginnt nun der Pflegeprozess. Zunächst wird der Chemikalienschutzanzug 10 durch Öffnen des dem Druckluftkompressor 36 zugeordneten Ventils mithilfe des Druckluftkompressors 36 unter einen Ausdehnungs- bzw. Formstabilitätsdruck gesetzt. Insoweit ist der Druckkompressor 36 auch Teil einer erfindungsgemäßen Formstabilitätsdruckeinrichtung. Der Formstabilitätsdruck ist ausreichend, so dass sich der Chemikalienschutzanzug 10 auf sein volles oder nahezu volles Volumen ausdehnt. Anschließend wird das dem Dampfgenerator 34 zugeordnete Ventil geöffnet und durch den Dampfgenerator 34 wird auf eine vorgegebene Temperatur temperierter Wasserdampf als Reinigungs- und Desinfektionsfluid über den ersten Pflegeadapter 24 in das Innere des Chemikalienschutzanzugs 10 eingeleitet, wo er sich unter anderen aufgrund des Formstabilitätsdrucks selbsttätig gleichmäßig verteilt. Die Steuereinrichtung kontrolliert dabei über den Drucksensor 46 und den Temperatursensor 48 Temperatur und Druck im Inneren des Chemikalienschutzanzugs 10 und regelt ggf. zur Erreichung eines jeweiligen Sollwerts nach. Soweit der Wasserdampf bei dem zu pflegenden Anzug eine für das Abtöten von Keimen ausreichende Temperatur haben darf, ist das Zuführen eines separaten Desinfektionsmittels nicht erforderlich. Anderenfalls kann dem Wasserdampf über die Dosierpumpe 42 aus dem Reservoir 44 ein beispielsweise chemisches Desinfektionsmittel zugemischt werden. Der Wasserdampf kondensiert an den Innenflächen des Chemikalienschutzanzugs 10 und das ablaufende Kondensat sammelt sich im Kopfbereich 14, wo es von der Absaugvorrichtung 56 über die Absaugleitung 58 abgesaugt und insbesondere über die Pumpe 40 abgeführt wird. Sofern dem Wasserdampf ein Desinfektionsmittel zugemischt wurde, wird anschließend in einem Spülvorgang noch einmal reiner Wasserdampf in das Innere des Chemikalienschutzanzugs geleitet und über die Pumpe 40 abgepumpt.

Nach dem Reinigungsprozess startet die Dichtigkeitsprüfung. Dazu wird der Chemikalienschutzanzug 10 durch den Druckluftkompressor 36 zunächst unter einen Stabilisierungsdruck gesetzt und der Stabilisierungsdruck wird während eines vorgegebenen Zeitintervalls gehalten. Anschließend wird der Chemikalienschutzanzug 10 durch den Druckluftkompressor 36 unter den höheren Prüfdruck gesetzt und das Leitungssystem wird durch Schließen der Ventile geschlossen. Der Drucksensor 46 überwacht für einen vorgegebenen Zeitraum den Druckabfall. Die Messergebnisse können beispielsweise von der Steuereinrichtung dokumentiert und protokolliert werden. Sofern der Druckabfall oberhalb eines Grenzwerts bleibt, wird die Dichtigkeitsprüfung durch Öffnen beispielsweise des der Abführleitung 52 zugeordneten Ventils beendet, so dass der Druck in dem Chemikalienschutzanzug 10 absinkt, beispielsweise auf den Formstabilitätsdruck. Wird im Rahmen der Dichtigkeitsprüfeinrichtung dagegen ein unzulässig hoher Druckabfall registriert, gibt die Steuereinrichtung ein Warnsignal aus und kann dieses Messergebnis ebenfalls protokollieren. In diesem Fall ist der Chemikalienschutzanzug nicht mehr weiter zu verwenden bzw. zu reparieren und vor einer Verwendung erneut zu prüfen.

Anschließend kann der Trocknungsprozess beginnen. Es ist aber auch möglich, dass die Trocknung vor der Dichtigkeitsprüfung erfolgt. Dazu wird durch das Heizgebläse 38 durch Öffnen des diesem zugeordneten Ventils warme Luft in das Innere des Chemikalienschutzanzugs 10 gebracht, wobei die warme Luft die Restfeuchtigkeit in dem Chemikalienschutzanzug 10 aufnimmt und diese über eine Rückführöffnung des ersten Pflegeadapters 24 und über die Abführleitung 50 abführt. Nach einem vorgegebenen Zeitraum wird der Trocknungsprozess beendet.

Bei Verwendung einer Pflegekabine kann in an sich bekannter Weise noch eine Außenreinigung, -desinfektion und -trocknung erfolgen, insbesondere vor oder nach dem oben erläuterten Pflegeprozess.

Bei Verwendung einer Pflegekabine wird der Chemikalienschutzanzug 10 anschließend aus dieser herausgefahren, wiederum beispielsweise automatisch durch die Steuereinrichtung gesteuert. Es kann nun der Reißverschluss des Chemikalienschutzanzugs 10 von einem Gerätewart geöffnet werden und der Gerätewart kann die Ablaufvorrichtung 56 und die Absaugleitung 58 aus dem Inneren des Kopfteils 14 entfernen und die Pflegeadapter 24, 26 von den Abluftventilöffnungen 22 lösen. Der Chemikalienschutzanzug 10 kann nun zur weiteren Verwendung entnommen werden.

## Patentansprüche

1. Verfahren zur Pflege mindestens eines Schutzanzugs (10), insbesondere eines Chemikalienschutzanzugs (10), umfassend die Schritte:
a) mindestens ein Pflegeadapter (24, 26) wird mit mindestens einer Öffnung des Schutzanzugs (10) lösbar und dichtend verbunden,
b) über den mindestens einen Pflegeadapter (24, 26) wird ein Reinigungs- und Desinfektionsfluid in das Innere des Schutzanzugs (10) geleitet und das in das Innere des Schutzanzugs (10) geleitete Reinigungs- und Desinfektionsfluid aus dem Schutzanzug (10) abgeführt,
c) über den mindestens einen Pflegeadapter (24, 26) wird ein Trocknungsgas zur Trocknung des Schutzanzugs (10) in das Innere des Schutzanzugs (10) geleitet und das in das Innere des Schutzanzugs (10) geleitete Trocknungsgas aus dem Schutzanzug (10) abgeführt,
d) der mindestens eine Pflegeadapter (24, 26) wird von der mindestens einen Öffnung des Schutzanzugs getrennt, **dadurch gekennzeichnet, dass** nach dem Verfahrensschritt b) und vor dem Verfahrensschritt c) in einem Verfahrensschritt b1) über den mindestens einen Pflegeadapter (24, 26) ein Druckgas zur Dichtigkeitsprüfung des Schutzanzugs (10) in das Innere des Schutzanzugs (10) geleitet wird bis in dem Schutzanzug (10) ein vorgegebenes Druckniveau herrscht, das in dem Schutzanzug (10) herrschende Druckniveau über einen vorgegebenen Zeitraum gemessen wird, und anschließend das in das Innere des Schutzanzugs (10) geleitete Druckgas über den mindestens einen Pflegeadapter (24, 26) aus dem Schutzanzug (10) abgeführt wird, und dass in dem Verfahrensschritt bl) weiterhin eine Warnmeldung ausgegeben wird, sofern das Druckniveau in dem vorgegebenen Zeitraum unter einen definierten Grenzwert fällt.

2. Verfahren nach Anspruch 1, **dadurch gekennzeichnet, dass** zumindest die Verfahrensschritte b) und c) von einer Steuereinrichtung automatisch durchgeführt werden.

3. Verfahren nach Anspruch **dadurch gekennzeichnet, dass** auch der Verfahrensschritt b1) von der Steuereinrichtung automatisch durchgeführt wird.

4. Verfahren nach einem der vorhergehenden Ansprüche, **dadurch gekennzeichnet, dass** die mindestens eine Öffnung mindestens eine Ventilöffnung, insbesondere mindestens eine Zuluftventilöffnung und/oder mindestens eine Abluftventilöffnung (22), des Schutzanzugs (10) ist.

5. Verfahren nach einem der vorhergehenden Ansprüche, **dadurch gekennzeichnet, dass** der Schutzanzug (10) zumindest während des Einleitens und des Abführens des Reinigungs- und Desinfektionsfluids durch Einleiten eines Druckgases unter einen die Formstabilität des Schutzanzugs (10) gewährleistenden Ausdehnungsdruck gesetzt wird.

6. Verfahren nach einem der vorhergehenden Ansprüche, **dadurch gekennzeichnet,**
- **dass** ein erster Pflegeadapter (24) mit einer ersten Öffnung des Schutzanzugs (10) lösbar und dichtend verbunden wird, und ein zweiter Pflegeadapter (26) mit einer zweiten Öffnung des Schutzanzugs (10) lösbar und dichtend verbunden wird,
- **dass** das Einleiten des Reinigungs- und Desinfektionsfluids über den ersten Pflegeadapter (24) und das Abführen des Reinigungs- und Desinfektionsfluids über den zweiten Pflegeadapter (26) erfolgt, und
- **dass** das Einleiten des Trocknungsgases über den ersten Pflegeadapter (24) oder den zweiten Pflegeadapter (26) und das Abführen des Trocknungsgases über den zweiten Pflegeadapter (26) oder den ersten Pflegeadapter (24) erfolgt.

7. Verfahren nach 6, **dadurch gekennzeichnet, dass** das Einleiten des Druckgases über den ersten Pflegeadapter (24) oder den zweiten Pflegeadapter (26) und das Abführen des Druckgases über den zweiten Pflegeadapter (26) oder den ersten Pflegeadapter (24) erfolgt.

8. Verfahren nach einem der vorhergehenden Ansprüche, **dadurch gekennzeichnet, dass** die Pflege des Schutzanzugs (10) in einer verschließbaren Pflegekabine erfolgt, wobei die lösbare und dichtende Verbindung des mindestens einen Pflegeadapters (24, 26) mit der mindestens einen Öffnung außerhalb der Pflegekabine erfolgt und der Schutzanzug (10) automatisch in die Pflegekabine verfahren wird.

9. System zur Pflege mindestens eines Schutzanzugs (10), insbesondere eines Chemikalienschutzanzugs (10) umfassend:
- mindestens einen Pflegeadapter (24, 26), der lösbar und dichtend mit mindestens einer Öffnung des Schutzanzugs (10) verbindbar ist,
- eine mit dem mindestens einen Pflegeadapter (24, 26) verbindbare Reinigungs- und Desinfektionsfluidzuführeinrichtung zum Einleiten eines Reinigungs- und Desinfektionsfluids in das Innere des Schutzanzugs (10) und zum Abführen des in das Innere des Schutzanzugs (10) geleiteten Reinigungs- und Desinfektionsfluids aus dem Schutzanzug (10),
- eine mit dem mindestens einen Pflegeadapter (24, 26) verbindbare Trocknungsgaszuführeinrichtung zum Einleiten eines Trocknungsgases in das Innere des Schutzanzugs (10) und zum Abführen des in das Innere des Schutzanzugs (10) geleiteten Trocknungsgases aus dem Schutzanzug (10),
- eine Steuereinrichtung zur Steuerung des Pflegeprozesses,
- **dadurch gekennzeichnet, dass** das System weiterhin eine mit dem mindestens einen Pflegeadapter (24, 26) verbindbare Dichtigkeitsprüfeinrichtung mit einer Druckmesseinrichtung umfasst, wobei die Dichtigkeitsprüfeinrichtung dazu ausgebildet ist, Druckgas zur Dichtigkeitsprüfung des Schutzanzugs (10) über den mindestens einen Pflegeadapter (24, 26) in das Innere des Schutzanzugs (10) einzuleiten bis in dem Schutzanzug (10) ein vorgegebenes Druckniveau herrscht, wobei die Druckmesseinrichtung dazu ausgebildet ist, das in dem Schutzanzug (10) herrschende Druckniveau über einen vorgegebenen Zeitraum zu messen, und wobei die Dichtigkeitsprüfeinrichtung dazu ausgebildet ist, das Druckgas nach Ablauf des vorgegebenen Zeitraums über den mindestens einen Pflegeadapter (24, 26) aus dem Schutzanzug (10) abzuführen, und dass die Dichtigkeitsprüfeinrichtung weiterhin dazu ausgebildet ist, eine Warnmeldung auszugeben, sofern das Druckniveau in dem vorgegebenen Zeitraum unter einen definierten Grenzwert fällt.

10. System nach Anspruch 9, **dadurch gekennzeichnet, dass** die Steuereinrichtung die Reinigungs- und Desinfektionsfluidzuführeinrichtung, und die Trocknungsgaszuführeinrichtung automatisch ansteuert.

11. System nach Anspruch 10, **dadurch gekennzeichnet, dass** die Steuereinrichtung auch die Dichtigkeitsprüfeinrichtung automatisch ansteuert.

12. System nach einem der Ansprüche 9 bis 11, **dadurch gekennzeichnet, dass** die mindestens eine Öffnung mindestens eine Ventilöffnung, insbesondere mindestens eine Zuluftventilöffnung und/oder mindestens eine Abluftventilöffnung (22), des Schutzanzugs (10) ist.

13. System nach einem der Ansprüche 9 bis 12, **dadurch gekennzeichnet, dass** es weiterhin eine Formstabilitätsdruckeinrichtung umfasst, die dazu ausgebildet ist, den Schutzanzug (10) zumindest während des Einleitens und des Abführens des Reinigungs- und Desinfektionsfluids durch Einleiten eines Druckgases unter einen die Formstabilität des Schutzanzugs (10) gewährleistenden Ausdehnungsdruck zu setzen.

14. System nach einem der Ansprüche 9 bis 13,
- wobei das System einen ersten Pflegeadapter (24) umfasst, der mit einer ersten Öffnung des Schutzanzugs (10) lösbar und dichtend verbindbar ist, und wobei das System einen zweiten Pflegeadapter (26) umfasst, der mit einer zweiten Öffnung des Schutzanzugs (10) lösbar und dichtend verbindbar ist,
- wobei die Reinigungs- und Desinfektionsfluidzuführeinrichtung derart mit dem ersten Pflegeadapter (24) und/oder dem zweiten Pflegeadapter (26) verbindbar ist, dass das Einleiten des Reinigungs- und Desinfektionsfluids über den ersten Pflegeadapter (24) und das Abführen des Reinigungs- und Desinfektionsfluids über den zweiten Pflegeadapter (26) erfolgt, und
- wobei die Trocknungsgaszuführeinrichtung derart mit dem ersten Pflegeadapter (24) und/oder dem zweiten Pflegeadapter (26) verbindbar ist, dass das Einleiten des Trocknungsgases über den ersten Pflegeadapter (24) oder den zweiten Pflegeadapter (26) und das Abführen des Trocknungsgases über den zweiten Pflegeadapter (26) oder den ersten Pflegeadapter (24) erfolgt.

15. System nach Anspruch 14, **dadurch gekennzeichnet, dass** die Dichtigkeitsprüfeinrichtung derart mit dem ersten Pflegeadapter (24) und/oder dem zweiten Pflegeadapter (26) verbindbar ist, dass das Einleiten des Druckgases über den ersten Pflegeadapter (24) oder den zweiten Pflegeadapter (26) und das Abführen des Druckgases über den zweiten Pflegeadapter (26) oder den ersten Pflegeadapter (24) erfolgt.

16. System nach einem der Ansprüche 9 bis 15, **dadurch gekennzeichnet, dass** es eine Pflegekabine zur Durchführung der Pflege des Schutzanzugs umfasst, wobei eine automatische Zuführeinrichtung vorgesehen ist, mit der der zu pflegende Schutzanzug (10) bei mit der mindestens einen Öffnung lösbar und dichtend verbundenem mindestens einen Pflegeadapter (24, 26) automatisch in die Pflegekabine verfahren werden kann.

## Claims

1. A method for maintaining at least one protective suit (10), in particular a chemical protective suit (10), comprising the steps:
a) at least one maintenance adapter (24, 26) is releasably and sealingly connected to at least one opening of the protective suit (10),
b) a cleaning and disinfecting fluid is conducted into the interior of the protective suit (10) via the at least one maintenance adapter (24, 26) and the cleaning and disinfecting fluid conducted into the interior of the protective suit (10) is discharged from the protective suit (10),
c) a drying gas is conducted into the interior of the protective suit (10) via the at least one maintenance adapter (24, 26) for drying the protective suit (10) and the drying gas conducted into the interior of the protective suit (10) is discharged from the protective suit (10),
d) the at least one maintenance adapter (24, 26) is disconnected from the at least one opening of the protective suit, **characterized in that**, after the method step b) and before the method step c) in a method step b1), for the tightness testing of the protective suit (10) a compressed gas is conducted into the interior of the protective suit (10) via the at least one maintenance adapter (24, 26) until a predetermined pressure level prevails in the protective suit (10), the pressure level prevailing in the protective suit (10) is measured over a predetermined time period and then the compressed gas conducted into the interior of the protective suit (10) is discharged from the protective suit (10) via the at least one maintenance adapter (24, 26), and **in that** in the method step b1) a warning signal is also output if the pressure level falls below a defined limit value in the predetermined time period.

2. The method according to claim 1, **characterized in that** at least the methods steps b) and c) are carried out automatically by a control apparatus.

3. The method according to claim 2, **characterized in that** the method step b1) is also automatically carried out by the control apparatus.

4. The method according to one of the preceding claims, **characterized in that** the at least one opening is at least one valve opening, in particular at least one air intake valve opening and/or at least one air outlet valve opening (22), of the protective suit (10).

5. The method according to one of the preceding claims, **characterized in that** at least during the introduction and the discharge of the cleaning and disinfecting fluid the protective suit (10) is placed under an expansion pressure ensuring the dimensional stability of the protective suit (10) by introducing a compressed gas.

6. The method according to one of the preceding claims, **characterized**
- **in that** a first maintenance adapter (24) is releasably and sealingly connected to a first opening of the protective suit (10), and a second maintenance adapter (26) is releasably and sealingly connected to a second opening of the protective suit (10),
- **in that** the introduction of the cleaning and disinfecting fluid takes place via the first maintenance adapter (24) and the discharge of the cleaning and disinfecting fluid takes place via the second maintenance adapter (26), and
- **in that** the introduction of the drying gas takes place via the first maintenance adapter (24) or the second maintenance adapter (26) and the discharge of the drying gas takes place via the second maintenance adapter (26) or the first maintenance adapter (24).

7. The method according to claim 6, **characterized in that** the introduction of the compressed gas takes place via the first maintenance adapter (24) or the second maintenance adapter (26) and the discharge of the compressed gas takes place via the second maintenance adapter (26) or the first maintenance adapter (24).

8. The method according to one of the preceding claims, **characterized in that** the maintenance of the protective suit (10) takes place in a closable maintenance booth, wherein the releasable and sealing connection of the at least one maintenance adapter (24, 26) to the at least one opening takes place outside the maintenance booth and the protective suit (10) is automatically moved into the maintenance booth.

9. A system for maintaining at least one protective suit (10), in particular a chemical protective suit (10), comprising:
- at least one maintenance adapter (24, 26) which can be releasably and sealingly connected to at least one opening of the protective suit (10),
- a cleaning and disinfecting fluid supply apparatus which can be connected to the at least one maintenance adapter (24, 26) for introducing a cleaning and disinfecting fluid into the interior of the protective suit (10) and for discharging from the protective suit (10) the cleaning and disinfecting fluid conducted into the interior of the protective suit (10),
- a drying gas supply apparatus which can be connected to the at least one maintenance adapter (24, 26) for introducing a drying gas into the interior of the protective suit (10) and for discharging from the protective suit (10) the drying gas conducted into the interior of the protective suit (10),
- a control apparatus for controlling the maintenance process,
- **characterized in that** the system also comprises a tightness testing apparatus having a pressure measuring apparatus which can be connected to the at least one maintenance adapter (24, 26), wherein, for the tightness testing of the protective suit (10), the tightness testing apparatus is configured to introduce compressed gas into the interior of the protective suit (10) via the at least one maintenance adapter (24, 26) until a predetermined pressure level prevails in the protective suit (10), wherein the pressure measuring apparatus is configured to measure the pressure level prevailing in the protective suit (10) over a predetermined time period, and wherein after the elapse of the predetermined time period the tightness testing apparatus is configured to discharge from the protective suit (10) the compressed gas via the at least one maintenance adapter (24, 26), and **in that** the tightness testing apparatus is also configured to output a warning signal if the pressure level falls below a defined limit value in the predetermined time period.

10. The system according to claim 9, **characterized in that** the control apparatus automatically activates the cleaning and disinfecting fluid supply apparatus and the drying gas supply apparatus.

11. The system according to claim 10, **characterized in that** the control apparatus also automatically activates the tightness testing apparatus.

12. The system according to one of claims 9 to 11, **characterized in that** the at least one opening is at least one valve opening, in particular at least one air intake valve opening and/or at least one air outlet valve opening (22), of the protective suit (10).

13. The system according to one of claims 9 to 12, **characterized in that** it also comprises a pressure apparatus for maintaining dimensional stability which is configured to place the protective suit (10) under an expansion pressure ensuring the dimensional stability of the protective suit (10) by introducing a compressed gas at least during the introduction and the discharge of the cleaning and disinfecting fluid.

14. The system according to one of claims 9 to 13,
- wherein the system comprises a first maintenance adapter (24) which can be releasably and sealingly connected to a first opening of the protective suit (10), and wherein the system comprises a second maintenance adapter (26) which can be releasably and sealingly connected to a second opening of the protective suit (10),
- wherein the cleaning and disinfecting fluid supply apparatus can be connected to the first maintenance adapter (24) and/or the second maintenance adapter (26) such that the introduction of the cleaning and disinfecting fluid takes place via the first maintenance adapter (24) and the discharge of the cleaning and disinfecting fluid takes place via the second maintenance adapter (26), and
- wherein the drying gas supply apparatus can be connected to the first maintenance adapter (24) and/or the second maintenance adapter (26) such that the introduction of the drying gas takes place via the first maintenance adapter (24) or the second maintenance adapter (26) and the discharge of the drying gas takes place via the second maintenance adapter (26) or the first maintenance adapter (24).

15. The system according to claim 14, **characterized in that** the tightness testing apparatus can be connected to the first maintenance adapter (24) and/or the second maintenance adapter (26) such that the introduction of the compressed gas takes place via the first maintenance adapter (24) or the second maintenance adapter (26) and the discharge of the compressed gas takes place via the second maintenance adapter (26) or the first maintenance adapter (24).

16. The system according to one of claims 9 to 15, **characterized in that** it comprises a maintenance booth for carrying out the maintenance of the protective suit, wherein an automatic supply apparatus is provided, the protective suit (10) to be maintained being able to be moved thereby automatically into the maintenance booth when the at least one maintenance adapter (24, 26) is releasably and sealingly connected to the at least one opening.

## Revendications

1. Procédé pour l'entretien d'au moins un vêtement de protection (10), en particulier d'un vêtement de protection contre les produits chimiques (10), comprenant les étapes :
a) au moins un adaptateur d'entretien (24, 26) est raccordé de façon amovible et étanche avec au moins une ouverture du vêtement de protection (10),
b) un fluide de nettoyage et de désinfection est introduit à l'intérieur du vêtement de protection (10) et le fluide de nettoyage et de désinfection introduit à l'intérieur du vêtement de protection (10) est évacué hors du vêtement de protection (10), par l'au moins un adaptateur d'entretien (24, 26),
c) un gaz de séchage est introduit à l'intérieur du vêtement de protection (10) pour le séchage du vêtement de protection (10) et le gaz de séchage introduit à l'intérieur du vêtement de protection (10) est évacué hors du vêtement de protection (10), par l'au moins un adaptateur d'entretien (24, 26),
d) l'au moins un adaptateur d'entretien (24, 26) est séparé de l'au moins une ouverture du vêtement de protection, **caractérisé en ce que**, pour la vérification de l'étanchéité du vêtement de protection (10), un gaz comprimé est introduit à l'intérieur du vêtement de protection (10) par l'au moins un adaptateur d'entretien (24, 26), après l'étape de procédé b) et avant l'étape de procédé c), lors d'une étape de procédé b1), jusqu'à ce qu'un niveau de pression spécifié soit donné dans le vêtement de protection (10), **en ce que** le niveau de pression donné dans le vêtement de protection (10) est mesuré pendant une période spécifiée, et ensuite, **en ce que** le gaz comprimé introduit à l'intérieur du vêtement de protection (10) est évacué hors du vêtement de protection (10) par l'au moins un adaptateur d'entretien (24, 26), et **en ce qu'**un message d'alerte est en outre émis si, dans la période spécifiée, le niveau de pression chute sous une valeur limite définie, lors de l'étape de procédé b1).

2. Procédé selon la revendication 1, **caractérisé en ce qu'**au moins les étapes de procédé b) et c) sont exécutées automatiquement par un dispositif de commande.

3. Procédé selon la revendication 2, **caractérisé en ce que** l'étape de procédé b1) aussi est exécutée automatiquement par le dispositif de commande.

4. Procédé selon l'une des revendications précédentes, **caractérisé en ce que** l'au moins une ouverture est au moins une ouverture de soupape du vêtement de protection (10), en particulier au moins une ouverture de soupape d'alimentation en air et/ou au moins une ouverture de soupape d'évacuation d'air (22).

5. Procédé selon l'une des revendications précédentes, **caractérisé en ce qu'**une pression de dilatation, garantissant la stabilité dimensionnelle du vêtement de protection (10), est appliquée au vêtement de protection (10) par l'introduction d'un gaz comprimé, au moins pendant l'introduction et l'évacuation du fluide de nettoyage et de désinfection.

6. Procédé selon l'une des revendications précédentes, **caractérisé**
- **en ce qu'**un premier adaptateur d'entretien (24) est raccordé de façon amovible et étanche à une première ouverture du vêtement de protection (10), et en ce qu'un deuxième adaptateur d'entretien (26) est raccordé de façon amovible et étanche à une deuxième ouverture du vêtement de protection (10),
- **en ce que** l'introduction du fluide de nettoyage et de désinfection est effectuée par le premier adaptateur d'entretien (24) et en ce que l'évacuation du fluide de nettoyage et de désinfection est effectuée par le deuxième adaptateur d'entretien (26), et
- **en ce que** l'introduction du gaz de séchage est effectuée par le premier adaptateur d'entretien (24) ou le deuxième adaptateur d'entretien (26), et en ce que l'évacuation du gaz de séchage est effectuée par le deuxième adaptateur d'entretien (26) ou par le premier adaptateur d'entretien (24).

7. Procédé selon la revendication 6, **caractérisé en ce que** l'introduction du gaz comprimé ets effectuée par le premier adaptateur d'entretien (24) ou le deuxième adaptateur d'entretien (26), et **en ce que** l'évacuation du gaz comprimé est effectuée par le deuxième adaptateur d'entretien (26) ou le premier adaptateur d'entretien (24).

8. Procédé selon l'une des revendications précédentes, **caractérisé en ce que** l'entretien du vêtement de protection (10) est effectué dans une cabine d'entretien pouvant être fermée, le raccordement amovible et étanche de l'au moins un adaptateur d'entretien (24, 26) à l'au moins une ouverture étant effectué hors de la cabine d'entretien, et **en ce que** le vêtement de protection (10) est transporté automatiquement dans la cabine d'entretien.

9. Système pour l'entretien d'au moins un vêtement de protection (10), en particulier d'un vêtement de protection contre les produits chimiques (10), comprenant :
- au moins un adaptateur d'entretien (24, 26), lequel est raccordé de façon amovible et étanche à l'au moins une ouverture du vêtement de protection (10),
- un dispositif d'introduction de fluide de nettoyage et de désinfection pouvant être raccordé à l'au moins un adaptateur d'entretien (24, 26) pour l'introduction à l'intérieur du vêtement de protection (10) d'un fluide de nettoyage et de désinfection et pour l'évacuation hors du vêtement de protection (10) du fluide de nettoyage et de désinfection introduit à l'intérieur du vêtement de protection (10),
- un dispositif d'introduction de gaz de séchage pouvant être raccordé à l'au moins un adaptateur d'entretien (24, 26) pour l'introduction à l'intérieur du vêtement de protection (10) d'un gaz de séchage et pour l'évacuation hors du vêtement de protection (10) du gaz de séchage introduit à l'intérieur du vêtement de protection (10),
- un dispositif de commande pour la commande du processus d'entretien, **caractérisé en ce que** le système comprend en outre un dispositif de vérification d'étanchéité avec un dispositif de mesure de pression, pouvant être raccordé avec l'au moins un adaptateur d'entretien (24, 26), le dispositif de vérification d'étanchéité étant conçu pour introduire à l'intérieur du vêtement de protection (10), par l'au moins un adaptateur d'entretien (24, 26), du gaz comprimé pour la vérification de l'étanchéité du vêtement de protection (10), jusqu'à ce qu'un niveau de pression spécifié soit donné dans le vêtement de protection (10), le dispositif de mesure de pression étant conçu pour mesurer pendant une période spécifiée le niveau de pression donné dans le vêtement de protection (10), et le dispositif de vérification d'étanchéité étant conçu pour évacuer le gaz comprimé hors du vêtement de protection (10) par l'au moins un adaptateur d'entretien (24, 26) après expiration de la période spécifiée, et **en ce que** le dispositif de vérification d'étanchéité est conçu en outre pour émettre un message d'alerte si, dans la période spécifiée, le niveau de pression chute sous une valeur limite définie.

10. Système selon la revendication 9, **caractérisé en ce que** le dispositif de commande pilote automatiquement le dispositif d'introduction de fluide de nettoyage et de désinfection, et le dispositif d'introduction de gaz de séchage.

11. Système selon la revendication 10, **caractérisé en ce que** le dispositif de commande pilote automatiquement le dispositif de vérification d'étanchéité aussi.

12. Système selon l'une des revendications 9 à 11, **caractérisé en ce que** l'au moins une ouverture est au moins une ouverture de soupape du vêtement de protection (10), en particulier au moins une ouverture de soupape d'alimentation en air et/ou au moins une ouverture de soupape d'évacuation d'air (22).

13. Système selon l'une des revendications 9 à 12, **caractérisé en ce qu'**il comprend en outre un dispositif de pression pour stabilité dimensionnelle, lequel est conçu pour appliquer au vêtement de protection (10) une pression de dilatation garantissant la stabilité dimensionnelle du vêtement de protection (10), au moins pendant l'introduction et l'évacuation du fluide de nettoyage et de désinfection, par l'introduction d'un gaz comprimé.

14. Système selon l'une des revendications 9 à 13,
- le système comprenant un premier adaptateur d'entretien (24), lequel peut être raccordé de façon amovible et étanche avec une première ouverture du vêtement de protection (10), et le système comprenant un deuxième adaptateur d'entretien (26), lequel peut être raccordé de façon amovible et étanche avec une deuxième ouverture du vêtement de protection (10),
- le dispositif d'introduction de fluide de nettoyage et de désinfection pouvant être raccordé avec le premier adaptateur d'entretien (24) et/ou le deuxième adaptateur d'entretien (26) de telle sorte que l'introduction du fluide de nettoyage et de désinfection est effectuée par le premier adaptateur d'entretien (24) et que l'évacuation du fluide de nettoyage et de désinfection est effectuée par le deuxième adaptateur d'entretien (26), et
- le dispositif d'introduction de gaz de séchage pouvant être raccordé avec le premier adaptateur d'entretien (24) et/ou le deuxième adaptateur d'entretien (26) de telle sorte que l'introduction du gaz de séchage est effectuée par le premier adaptateur d'entretien (24) ou le deuxième adaptateur d'entretien (26) et que l'évacuation du gaz de séchage est effectuée par le deuxième adaptateur d'entretien (26) ou le premier adaptateur d'entretien (24).

15. Système selon la revendication 14, **caractérisé en ce que** le dispositif de vérification d'étanchéité peut être raccordé avec le premier adaptateur d'entretien (24) et/ou le deuxième adaptateur d'entretien (26) de telle sorte que l'introduction du gaz comprimé est effectuée par le premier adaptateur d'entretien (24) ou le deuxième adaptateur d'entretien (26), et **en ce que** l'évacuation du gaz comprimé est effectuée par le deuxième adaptateur d'entretien (26) ou le premier adaptateur d'entretien (24).

16. Système selon l'une des revendications 9 à 15, **caractérisé en ce qu'**il comprend une cabine d'entretien pour l'exécution de l'entretien du vêtement de protection, un dispositif d'amenée automatique étant prévu, avec lequel le vêtement de protection (10) devant être entretenu peut être transporté automatiquement dans la cabine d'entretien par au moins un adaptateur d'entretien (24, 26) raccordé de façon amovible et étanche à l'au moins une ouverture.
